# EUROPEAN PATENT APPLICATION

(11) **EP 4 527 339 A1**
(43) Date of publication of application: **26.03.2025**
(21) Application number: 22942115.1
(22) Date of filing: 20.05.2022
(51) Int. Cl.: A61B 34/37

(54) **SURGICAL DEVICE, SURGICAL METHOD, AND SURGICAL APPARATUS**

(71) Applicant: Beijing Great Robotics Technology Limited, Beijing 100015 (CN); Suzhou Great Robotics Medical Technology Co., Ltd., Suzhou, Jiangsu 215024 (CN)
(72) Inventor: PAN, Wenrong, Beijing 100015 (CN)
(74) Representative: Dai, Simin
(86) International application number: PCT/CN2022/094031
(87) International publication number: WO 2023/221075

(57) **Abstract**

The specification discloses a surgical device, a surgical method and a surgical apparatus, the surgical device includes a first auxiliary mechanical arm (1), an operation mechanical arm (2) and a control device (3), the control device (3) sends control instructions to the first auxiliary mechanical arm (1) and the operation mechanical arm (2) according to positions of the first auxiliary mechanical arm (1) and the operation mechanical arm (2) and an operation action of an operator, such that a working cannula (13) disposed on a securing apparatus (11) configured at a distal end of the first auxiliary mechanical arm (1) rotates to push a dangerous part around a pre-planned designated area (7) away, and the operation mechanical arm (2), in response to the control instructions sent by the control device (3), controls a surgical instrument (211) included in the surgical instrument assembly (21) to perform surgical operation on the designated area (7). It can be seen that controlling the first auxiliary mechanical arm (1) to automatically rotate the working cannula (13) can protect the human tissue in the dangerous area, thereby reducing the risk of intraoperative infection and postoperative complications, and avoiding the manual rotation operation performed by the operator in the operation.

## Description

### TECHNICAL FIELD

This specification relates to the field of large surgical procedures, and in particular, to a surgical device, a surgical method, and a surgical apparatus.

### BACKGROUND

With the development of minimally invasive surgery technology, the requirements for minimally invasive and accurate surgery are increasing. Spinal endoscopic minimally invasive surgery, as an earlier minimally invasive technology, has been widely used clinically. Compared with the traditional open surgery, the spinal endoscope minimally invasive surgery has the advantages of small trauma, less bleeding, fast recovery, exact curative effect and the like.

In the related art, in order to reduce the difficulty of manual operation of an operator, the surgical device can be used to assist the operator in performing surgical operation, such that the uncertainty of manual operation in the surgical operation is reduced, and the burden of the operator in the operation is relieved while the safety of the operation is improved.

However, the current surgical device still tends to pay attention to the accuracy of the surgical instrument reaching the diseased part, and the protection operation on other parts around the diseased part still needs to be manually performed by an operator, which leads to the continued occurrence of intraoperative accidental injuries, infections, and postoperative complications, and the actual probability of occurrence is relatively high.

### SUMMARY

The specification provides a surgical device, a surgical method, and a surgical apparatus to partially solve the above problems in the related art.

The specification adopts the following technical solutions.

The specification provides a surgical device including a first auxiliary mechanical arm 1, an operation mechanical arm 2, and a control device 3;
where, the control device 3 is configured to acquire a position of the first auxiliary mechanical arm 1 and a position of the operation mechanical arm 2, and send control instructions to the first auxiliary mechanical arm 1 and the operation mechanical arm 2 according to the position of the first auxiliary mechanical arm 1, the position of the operation mechanical arm 2 and an operation action of an operator;
the first auxiliary mechanical arm 1 includes a securing apparatus 11 and a rotating apparatus 12; the rotating apparatus 12 is on the securing apparatus 11; the securing apparatus 11 is used for placing a working cannula 13 inserted into a patient; in response to a control instruction sent by the control device 3, the working cannula 13 placed on the securing apparatus 11 is controlled to rotate and push away a dangerous part around a pre-planned designated area 7 in a puncture wound; and
the operation mechanical arm 2 is configured with a surgical instrument assembly 21; and the operation mechanical arm 2 is configured to control, in response to a control instruction sent by the control device 3, a surgical instrument 211 included in the surgical instrument assembly 21 to perform a surgical operation on the designated area 7.

Optionally, the surgical device further includes a second auxiliary mechanical arm 4; the second auxiliary mechanical arm 4 includes a securing needle 41;
when determining that the patient is at a designated position, the control device 3 configured to control the second auxiliary mechanical arm 4 to press the patient;
the second auxiliary mechanical arm 4 configured to control, in response to a first puncture instruction sent by the control device 3, the securing needle 41 configured at a distal end of the second auxiliary mechanical arm 4 to puncture a body of the patient, so as to secure a diseased part of the patient.

Optionally, the surgical device further includes a second auxiliary mechanical arm 4; and the second auxiliary mechanical arm 4 includes a securing needle 41;
when the patient is at the designated position, the second auxiliary mechanical arm 4 is manually controlled by the operator to press the patient; and
the securing needle 41 configured at a distal end of the second auxiliary mechanical arm 4 is manually controlled by the operator to puncture the patient, so as to secure the diseased part of the patient.

Optionally, the surgical device further includes a scanning device 5; the scanning device 5 is configured to scan the patient and obtain a scanning result of the diseased part in the body of the patient; and
the scanning device 5 is configured to send the scanning result of the diseased part to the control device 3, such that the control device 3 receives and displays the scanning result of the diseased part.

Optionally, the surgical device further includes a positioning device 6; the operation mechanical arm 2 is configured with a first positioning light source mark 22; the first auxiliary mechanical arm 1 is configured with a second positioning light source mark 17; the second auxiliary mechanical arm 4 is configured with a third positioning light source mark 42; and
the positioning device 6 is configured to determine a relative position of the operation mechanical arm 2 relative to the patient and a relative position of the first auxiliary mechanical arm 1 relative to the patient according to the collected light emitted or reflected by the first positioning light source mark 22, the second positioning light source mark 17 and the third positioning light source mark 42, and send the relative positions to the control device 3, such that the control device 3 sends the control instructions to the first auxiliary mechanical arm 1 and the operation mechanical arm 2 according to the positions of the operation mechanical arm 2 and the first auxiliary mechanical arm 1 and the operation action of the operator.

Optionally, the control device 3 includes a planning module 31; the planning module 31 is configured to determine the designated area 7, a puncture position and a puncture direction 8 according to the positions of the operation mechanical arm 2 and the first auxiliary mechanical arm 1 and the scanning result of the diseased part, where the designated area 7 is an area allowing the surgical instrument 211 configured at the distal end of the operation mechanical arm 2 to perform a safe surgical operation on the diseased part, and the puncture position and the puncture direction 8 are used for enabling the working cannula 13 and the surgical instrument 211 to reach the designated area 7, such that the control device 3 controls the first auxiliary mechanical arm 1 and the operation mechanical arm 2 to perform a surgical operation on the designated area according to the designated area 7, the puncture position and the puncture direction 8.

Optionally, the first auxiliary mechanical arm 1 includes a first slide rail 15 and a clamping apparatus 14; where the first slide rail 15 is fixed on the first auxiliary mechanical arm 1; the clamping apparatus 14 is configured with a first slide block 141, and the first slide block 141 is configured to slide on the first slide rail 15 to drive the clamping apparatus 14 to slide on the first auxiliary mechanical arm 1 along an extension direction of the first slide rail 15, so as to drive a tool clamped by the clamping apparatus 14 to slide along the extension direction of the first slide rail 15.

Optionally, the clamping apparatus 14 is configured to clamp a puncture tool 16; and the clamping apparatus 14 is configured to control, in response to a second puncture instruction sent by the control device 3, the first slide block 141 to slide along the puncture direction 8 on the first slide rail 15, so as to drive the puncture tool 16 to puncture along the puncture direction 8 at the puncture position, such that a head end of the puncture tool 16 reaches the designated area 7 to form the puncture wound.

Optionally, the working cannula 13 is inserted into the puncture wound along the puncture direction 8 and secured on the securing apparatus 11, such that a head end of the working cannula 13 is in the designated area 7; and
when it is detected that the head end of the working cannula 13 is in the designated area 7, in response to a rotation instruction sent by the control device 3, the rotating apparatus 12 is controlled to rotate and drive the working cannula 13 placed on the securing apparatus 11 to rotate, such that the head end of the working cannula 13 pushes away a dangerous part around the designated area 7 in the puncture wound.

Optionally, the rotation instruction carries a designated rotation direction and a designated rotation angle of the working cannula 13; and the designated rotation direction and the designated rotation angle are determined by the control device 3 according to the designated area 7 and a scanning result of the diseased part.

Optionally, the clamping apparatus 14 is configured to clamp an endoscope 9; the clamping apparatus 14 is configured to control, in response to an endoscope control instruction sent by the control device 3, the first slide block 141 to slide along the first slide rail 15 to drive the endoscope 9 clamped by the clamping apparatus 14 to be inserted into the working cannula 13, so as to collect and send a real-time image of the designated area 7.

Optionally, the operation mechanical arm 2 includes a second slide rail 23 and a surgical instrument assembly 21; where the second slide rail 23 is fixed on the operation mechanical arm 2; the surgical instrument assembly 21 is configured with a second slide block 24, and the second slide block 24 is configured to slide on the second slide rail 23 to drive the surgical instrument assembly 21 to slide on the operation mechanical arm 2 along an extension direction of the second slide rail 23, so as to insert the surgical instrument 211 into the working cannula 13 or a working channel of the endoscope 9, to perform the surgical operation on the designated area 7.

Optionally, the surgical instrument assembly 21 includes a plurality of surgical instruments 211; and the plurality of surgical instruments 211 are mounted in the surgical instrument assembly 21 in a form including at least one of: parallel mounting, staggered mounting, inclined surface mounting, and zigzag mounting; and
the surgical instrument assembly 21 is configured to control, in response to an instrument switching instruction sent by the control device 3, switching between the surgical instruments 211 included in the surgical instrument assembly 21.

Optionally, the control device 3 includes a display 32, an auxiliary control lever 33 and an operation control lever 34;
where the display 32 is configured to display a real-time image of the designated area 7 and the scanning result of the diseased part;
the auxiliary control lever 33 is configured to send a rotation instruction to the rotating apparatus 12 in response to a first operation action of an operator, such that the rotating apparatus 12 rotates to drive the working cannula 13 placed on the securing apparatus 11 to rotate, and push away a dangerous part around the designated area 7 in the puncture wound; and
the operation control lever 34 is configured to, in response to a second operation action of the operator, send an instrument movement instruction to the operation mechanical arm 2 and control the second slide block 24 configured on the surgical instrument assembly 21 to slide along the second slide rail 23 to drive the surgical instrument 211 included in the surgical instrument assembly 21 to be inserted into the working cannula 13 along the puncture direction 8; and when it is detected that the surgical instrument 211 reaches the designated area 7, the operation control lever 34 is configured to send a surgical operation instruction to the surgical instrument 211 in response to a third operation action of the operator, so as to control the surgical instrument 211 to perform a surgical operation on the designated area 7.

The specification provides a surgical method applied to a control device, including:
acquiring a position of the first auxiliary mechanical arm and a position of the operation mechanical arm;
when it is determined that a working cannula configured on the first auxiliary mechanical arm is inserted into a pre-planned designated area, in response to an operation action of an operator, sending a rotation instruction to the first auxiliary mechanical arm configured on any one of the foregoing surgical devices, such that a rotating apparatus configured on the first auxiliary mechanical arm controls the working cannula secured on the securing apparatus to rotate and push away a dangerous part around the pre-planned designated area in a puncture wound; and
when it is determined that the surgical instrument mounted on the operation mechanical arm is inserted into the puncture wound on a body of a patient, in response to the operation action of the operator, sending a surgical operation instruction to the operation mechanical arm configured on any one of the foregoing surgical devices, such that a surgical instrument assembly configured on the operation mechanical arm controls the surgical instrument included in the surgical instrument assembly to perform the surgical operation on the pre-planned designated area.

The present specification provides a surgical apparatus applied to a control device, including:
a position acquisition module, configured to acquire a position of the first auxiliary mechanical arm and a position of the operation mechanical arm;
a rotation instruction sending module, configured to: when it is determined that a working cannula configured on the first auxiliary mechanical arm is inserted into a pre-planned designated area, send, in response to an operation action of an operator, a rotation instruction to the first auxiliary mechanical arm configured on any one of the foregoing surgical devices, such that a rotation apparatus configured on the first auxiliary mechanical arm controls a working cannula secured on a securing apparatus to rotate, such that the working cannula pushes away a dangerous part around a pre-planned designated area in a puncture wound; and
a surgical operation instruction sending module, configured to: when it is determined that a surgical instrument mounted on the operation mechanical arm is inserted into the puncture wound on a body of a patient, send, in response to the operation action of the operator, a surgical operation instruction to the operation mechanical arm configured on any one of the foregoing surgical devices, such that a surgical instrument assembly configured on the operation mechanical arm controls a surgical instrument included in the surgical instrument assembly to perform a surgical operation on the pre-planned designated area.

The present disclosure provides a computer-readable storage medium storing computer programs, when executed by a processor, causing the processor to implement the above-mentioned surgical method.

The at least one technical solution described above adopted in the specification can achieve the following beneficial effects.

The specification provides a surgical device, the surgical device includes a first auxiliary mechanical arm, an operation mechanical arm and a control device, where the control device sends control instructions to the first auxiliary mechanical arm and the operation mechanical arm according to a position of the first auxiliary mechanical arm, a position of the operation mechanical arm and an operation action of an operator, such that a working cannula placed on a securing apparatus configured at a distal end of the first auxiliary mechanical arm rotates to push away a dangerous part around a pre-planned designated area in a wound, and further, the operation mechanical arm controls, in response to a control instruction sent by the control device, a surgical instrument included in the surgical instrument assembly to perform surgical operation on the designated area. It can be seen that by automatically rotating the working cannula by the surgical device, the human tissue in the dangerous area can be protected, thereby reducing the risk of intraoperative infection and postoperative complications, and eliminating the manual rotation operation performed by the operator in the surgical operation.

### BRIEF DESCRIPTION OF DRAWINGS

The drawings described here are used to provide a further understanding of the specification and constitute a part of the specification. The schematic embodiments of the specification and the description thereof are used to explain the specification, and do not constitute an improper limitation on the specification. Among the drawings:
FIG. 1 is a schematic diagram of a surgical device in the specification;
FIG. 2 is a schematic diagram of a surgical device in the specification;
FIG. 3 is a schematic diagram of a surgical device in the specification;
FIG. 4A is a schematic diagram of an operation manner of a surgical device in the specification;
FIG. 4B is a schematic diagram of an operation manner of a surgical device in the specification;
FIG. 4C is a schematic diagram of an operation manner of a surgical device in the specification;
FIG. 5A is a schematic diagram of an operation manner of a surgical device in the specification;
FIG. 5B is a schematic diagram of a surgical instrument arrangement in the specification;
FIG. 5C is a schematic diagram of a surgical instrument arrangement in the specification;
FIG. 6A is a schematic diagram of a surgical device in the specification;
FIG. 6B is a schematic diagram of a surgical device in the specification;
FIG. 7 is a schematic diagram of a surgical device in the specification;
FIG. 8 is a schematic diagram of a surgical method in the specification;
FIG. 9 is a schematic diagram of a surgical apparatus provided in the specification.

### DETAILED DESCRIPTION

In order to make the purpose, the technical solution, and the advantages of the specification clearer, the technical solution of the specification will be described clearly and comprehensively with reference to specific embodiments of the specification and corresponding drawings. Obviously, the described embodiments are only a part, but not all, of the embodiments of the specification. Based on the embodiments in the specification, all other embodiments obtained by a person of ordinary skill in the art without creative efforts shall fall within the scope of protection of the specification.

In addition, it should be noted that all actions of obtaining signals, information or data in the present disclosure are performed under the premise of complying with the corresponding data protection regulation policy of the country where the location is located, and obtaining authorization given by the corresponding device owner.

At present, whether it is orthopedics, neurology, or interventional procedures, minimally invasive surgery is required for treatment, the requirements for minimally invasive and accurate surgical operations are increasing, and it is necessary to achieve minimal trauma and accurately reach the affected area for effective treatment. The spinal endoscopic minimally invasive technology, as an earlier minimally invasive technology, has the advantages of minimal damage to the paraspinal muscles, maximum preservation of the complete spinal structure, and maintenance of postoperative spinal stability. It has the advantages of minimal trauma, less bleeding, fast recovery, fewer complications, and precise therapeutic effects.

At present, the use of minimally invasive surgical device to assist operators (such as doctors) in surgical operations has been widely used in the field of spinal endoscopic minimally invasive surgery. There are two main surgical methods commonly used for spinal endoscopic minimally invasive surgery, which are Percutaneous Endoscopic Lumbar Dissection (PELD) and Unilateral Biportal Endoscopy (UBE), in which the minimally invasive surgical device can perform auxiliary operation. In the spinal endoscopic minimally invasive surgery, due to the possibility of touching human tissues in dangerous areas such as the spinal cord and nerve roots when handling protruding intervertebral disc tissue in the diseased area, thus, in addition to conventional endoscopic placement and surgical instrument operations, it is also necessary to protect human tissues in the dangerous area during surgery. At present, a conventional method is to manually rotate a working cannula during a surgery, such that an open slope of the working cannula sequentially points to a head side, a back side, a tail side, and a ventral side, and a nerve root and a dural membrane are placed on the back side of the working channel for protective shielding, so as to avoid damage to human tissues (such as nerve roots) in a dangerous area when a minimally invasive surgical device assists an operator in performing a surgical operation on the diseased area, thereby causing complications.

However, the fundamental purpose of using a minimally invasive surgical device to assist the operator in performing a surgical operation is to reduce the burden of the operator on intraoperative operation, and currently, the operation of rotating the working cannula intraoperatively is still manually operated by the operator. Therefore, the specification provides a surgical device having a function of rotating a working cannula, such that the working cannula is automatically rotated by the surgical device during surgery to achieve the purpose of protecting human tissues in a dangerous area, thereby reducing the risk of intraoperative infection and postoperative complications.

The technical solutions provided by the embodiments of the specification will be described in detail below with reference to the drawings.

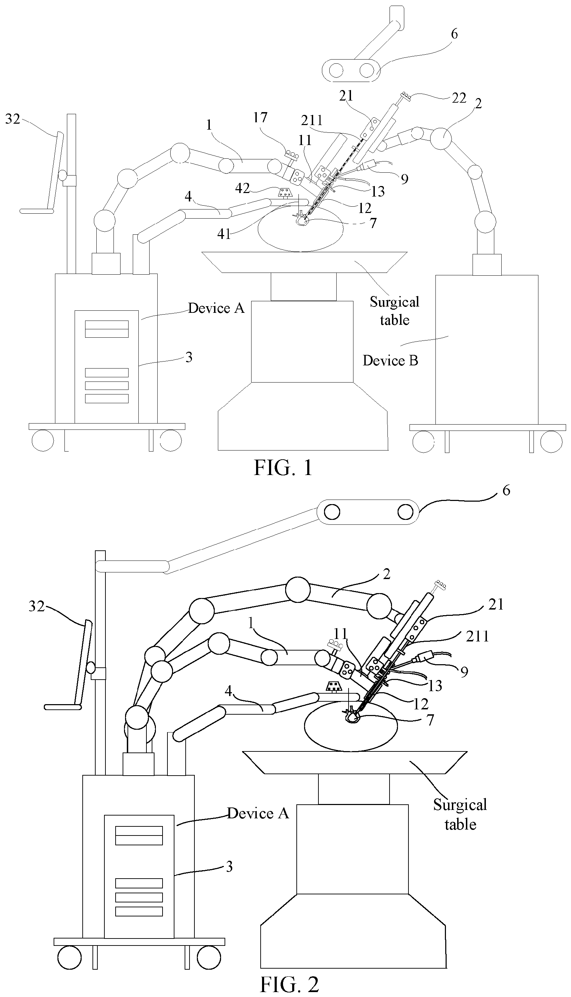

FIG. 1 is a schematic diagram of a surgical device provided by the specification;

The specification provides a surgical device that can assist an operator in performing a surgical operation. In the embodiments of the specification, for ease of understanding, a specific technical solution is described by using a spinal endoscopic surgery as an example.

As shown in FIG. 1, the surgical device includes at least a first auxiliary mechanical arm 1, an operation mechanical arm 2, and a control device 3. The device A and the device B are respectively on two sides of the surgical table, and the first auxiliary mechanical arm 1 is fixed on the device A, and the operation mechanical arm 2 is fixed on the device B. Typically, the patient is in a prone position in a designated position of the surgical table, such that the operator and the surgical device perform a surgical operation on the diseased part of the patient.

The first auxiliary mechanical arm 1 includes a securing apparatus 11 and a rotating apparatus 12 configured on the securing apparatus 11, where the securing apparatus 11 is used for placing (clamping) the working cannula 13, and the rotating apparatus 12 is used for rotating the working cannula 13 in response to a rotating instruction. To ensure flexibility of the first auxiliary mechanical arm 1, the first auxiliary mechanical arm 1 may be a mechanical arm having five or more movable joints.

The operation mechanical arm 2 includes a surgical instrument assembly 21 and a second slide rail 23, and the surgical instrument assembly 21 may include one or more surgical instruments 211 for minimally invasive spinal surgery, such as a nuclear forceps, an electrode electrocoagulation, a microscalpel, a microforceps, a microwave ablation needle, and the like. The surgical instrument assembly 21 is configured with a second slide block 24, and in response to an instrument movement instruction sent by the control device 3, the second slide block 24 slides on the second slide rail 23, to drive the surgical instrument assembly 21 to slide on the operation mechanical arm 2 along an extension direction (a puncture direction 8) of the second slide rail 23, to insert the surgical instrument 211 into the working cannula 13 or into a working channel of the endoscope 9, such that the surgical instrument 211 performs a surgical operation on the pre-planned designated area 7. The designated area 7 refers to a safe surgical area where a surgical operation can be performed in a diseased part of a patient. In order to ensure the flexibility of the operation mechanical arm 2, the operation mechanical arm 2 may be a mechanical arm having six or more than six movable joints. It should be noted that before the surgical operation is performed on the designated area 7 of the patient, the control device 3 may swing the first auxiliary mechanical arm 1 and the operation mechanical arm 2 to a surgical position suitable for performing the surgical operation on the patient according to the positions of the first auxiliary mechanical arm 1 and the operation mechanical arm 2 and the relative positions of the first auxiliary mechanical arm 1 and the operation mechanical arm 2 relative to the patient, where the surgical position suitable for performing the surgical operation on the patient may refer to: a surgical position suitable for the puncture tool 16 puncturing at the puncture position along the puncture direction 8, a surgical position suitable for the endoscope 9 placing into the working cannula 13 along the puncture direction, and a surgical position suitable for placing a surgical instrument 211 into the working cannula 13 along the puncture direction 8 or inserting the surgical instrument 211 into the working channel of the endoscope 9 to reach the designated area 7.

The control device 3 may be a computer host installed with a specified software (for example, a three-dimensional software, a planning software, etc.), and is connected to the first auxiliary mechanical arm 1, the operation mechanical arm 2 and other devices in a wired or wireless manner. The control device 3 may be configured inside the device A or inside the device B, or may be configured in a device C independent of the device A and the device B, which is not limited in the embodiments of the specification.

Optionally, the first auxiliary mechanical arm 1 and the operation mechanical arm 2 may also be mounted on the same device on one side of the hospital bed, and the device may be temporarily fixed on the ground, or the device may be configured with pulleys capable of moving and move according to actual requirements, as shown in FIG. 2.

In this embodiment, the surgical device further includes a positioning device 6, and the positioning device 6 may be separately suspended above the patient (above the surgical table), or may be mounted on the device A or the device B where the mechanical arm is located, or may be provided with a device C that is independent of the device A or the device B, specially used for mounting the positioning device 6, and placed above the patient, so as to obtain light emitted or reflected by the first positioning light source mark 22, the second positioning light source mark 17, or the third positioning light source mark 42.

The positioning light source mark may be an infrared light emitting device, a light emitting diode (LED), a passive reflection sphere, or the like, and the positioning device 6 captures the positions of the first auxiliary mechanical arm 1 and the operation mechanical arm 2 by receiving the light emitted or reflected by the positioning light source mark.

Specifically, in order to obtain the relative position between the first auxiliary mechanical arm 1 and the diseased part of the patient, and the relative position of the operation mechanical arm 2 and the diseased part of the patient, the control device 3 simulates the coordinate positions and shapes of the endoscope 9, the surgical instrument 211 mounted on the operation mechanical arm 2 and the diseased part of the patient in the three-dimensional image, the first auxiliary mechanical arm 1 is mounted with a second positioning light source mark 17, the operation mechanical arm 2 is mounted with a first positioning light source mark 22, and the second auxiliary mechanical arm 4 or a target position within a preset range of the diseased part of the patient is configured with a third positioning light source mark 42, so as to ensure that accurate coordinate positions and shapes of the endoscope 9 and the surgical instrument 211 in the three-dimensional image of the diseased part can be simulated. The specific position of the first positioning light source mark 22 on the operation mechanical arm 2, the specific position of the second positioning light source mark 17 on the first auxiliary mechanical arm 1 and the specific position of the third positioning light source mark 42 on the second auxiliary mechanical arm 4 or the target position of the third positioning light source mark 42 within the preset range of the diseased part of the patient may be set (or configured) according to a specific surgical scenario, which is not limited in the present specification.

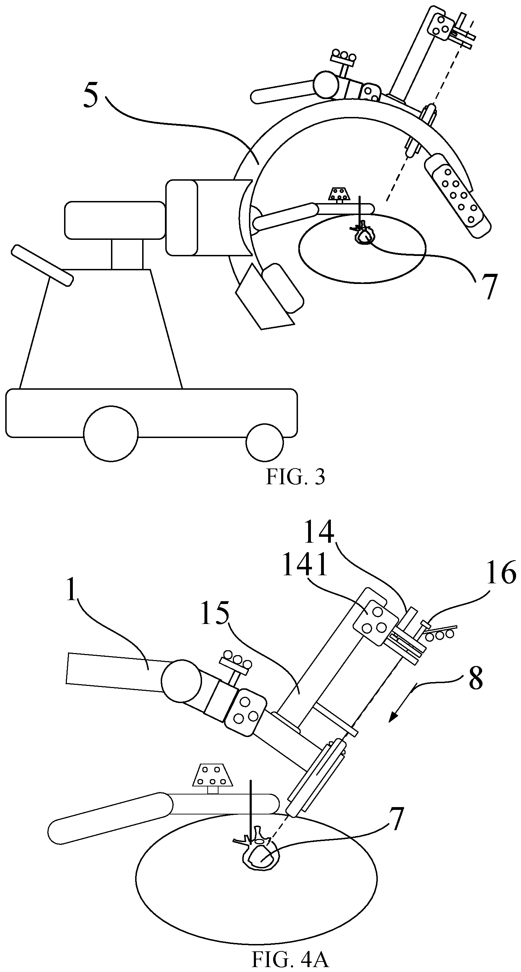

After the first positioning light source mark 22, the second positioning light source mark 17, and the third positioning light source mark 42 are deployed, the diseased part of the patient can be scanned through a scanning device 5 (for example, an intraoperative computed tomography (CT), a three-dimensional (3D) C-arm X-ray machine, etc.) provided in the surgical environment, as shown in FIG. 3. The scanning device 5 sends a scanning result to the control device 3, and then simulates three-dimensional images of the endoscope 9, the surgical instrument 211 and the diseased part of the patient through the three-dimensional software in the control device 3, and uploads the three-dimensional images to the display 32 on the control device 3 to provide reference for the surgical operation of the operator.

The control device 3 may plan an appropriate puncture position and a puncture direction 8 through a built-in planning module 31 (not shown in the figure) according to the position of the first auxiliary mechanical arm 1 and the position of the operation mechanical arm 2 determined by the positioning device 6 and the scanning result of the diseased part, so as to minimize trauma to the body of the patient while ensuring safety.

In addition, when a patient is operated, an area (such as nerve roots, spinal cord, etc. ) near the diseased part of the patient is often very dangerous, and once an operation error occurs and it is possible to cause other complications of the patient and even threaten the life safety of the patient, the control device 3 may plan, according to the position of the first auxiliary mechanical arm 1 and the position of the operation mechanical arm 2 determined by the positioning device 6, a safe operation area allowing the surgical instrument 211 to perform a surgical operation on the diseased part as the designated area 7, and a surgical operation is performed on the designated area 7, such that the surgical operation is performed on the diseased part on the body of the patient, because the surgical instrument 211 is mounted on the operation mechanical arm 2, the operation mechanical arm 2 can only move within a specified range and angle, ensuring that the surgical instrument 211 on the operation mechanical arm 2 cannot leave the designated area 7, thereby further ensuring the safety of the surgical operation.

Optionally, on the basis of the planned designated area 7, a dangerous area may also be planned according to the position of the first auxiliary mechanical arm 1 and the position of the operation mechanical arm 2 determined by the positioning device 6, where the dangerous area is an area where the surgical instrument 211 is not allowed to perform a surgical operation, and since the operation mechanical arm 2 can only move within a specified range and angle, it may be ensured that the surgical instrument 211 performs a surgical operation only in the designated area 7, and does not enter the dangerous area.

Optionally, the second auxiliary mechanical arm 4 may be mounted not only on the device A, but also on the device B or the surgical bed, or even on a separate device.

In addition, in order to keep away from a dangerous area around the diseased part when using surgical device to assist an operator (such as a doctor) in minimally invasive surgery, the rotation or tilting of the working cannula 13 placed on the first auxiliary mechanical arm 1 can be used to push away human tissues in the dangerous area around the diseased part, expanding the operating range of surgical instrument 211, and avoiding injury to the human tissue in the dangerous area caused by surgical operation of the surgical instrument 211 on the diseased area, resulting in intraoperative infection and postoperative complications..

In the embodiments of the specification, the operation steps of the control device 3 for the diseased part of the patient before the surgery specifically include the following three steps.

At first step: the control device 3 receives the relative position of the first auxiliary mechanical arm 1 relative to the patient and the relative position of the operating mechanical arm 2 relative to the patient acquired and sent by the positioning device 6.

At second step: the planning module 31 in the control device 3 determines, according to the relative positions of the first auxiliary mechanical arm 1 and the operating mechanical arm 2 relative to the patient and the scanning result of the diseased part of the patient, the designated area 7 that allows the surgical instrument 211 configured at the distal end of the operating mechanical arm 2 to perform a safe surgical operation on the diseased part, and a puncture position and a puncture direction 8 that enable the working cannula 13 and the surgical instrument 211 to reach the designated area 7. The puncture position and the puncture direction 8 that enable the surgical instrument 211 to reach the designated area 7 may serve as a surgical path planned by the control device 3 for the diseased part of the patient.

At third step: the control device 3 may send control instructions to the first auxiliary mechanical arm 1 and the operation mechanical arm 2 according to the determined surgical path planned for the diseased part and the operation action of the operator, such that the first auxiliary mechanical arm 1 and the operation mechanical arm 2, in response to a control instruction sent by the control device 3, take actions such as rotation and surgical operation.

In the embodiments of the specification, the first auxiliary mechanical arm 1 may perform, in response to a control instruction sent by the control device 3, pre-operative assistance and intra-operative assistance during the process of operating the operation mechanical arm 2 to perform the surgical operation on the diseased part of the patient. The pre-operative assistance may include puncturing, mounting the working cannula 13, and rotating or tilting the working cannula 13; and the intra-operative assistance may include rotating or tilting the working cannula 13.

Specifically, the first auxiliary mechanical arm 1 may include a first slide rail 15 and a clamping apparatus 14. The first slide rail 15 is fixed on the first auxiliary mechanical arm 1, the clamping apparatus 14 is provided with a first slide block 141, and the first slide block 141 slides on the first slide rail 15 to drive the clamping apparatus 14 to slide on the first auxiliary mechanical arm 1, so as to drive a tool clamped on the clamping apparatus 14, such as a puncture tool 16 and an endoscope 9. The following describes in detail the process of clamping the puncture tool 16 and the endoscope 9 by the clamping apparatus 14.

For the process of using the clamping apparatus 14 to clamp the puncture tool 16 to puncture the patient: as shown in FIG. 4A, when the patient is at the designated position and the first auxiliary mechanical arm 1 swings to the above side of the patient, the clamping apparatus 14 clamps the puncture tool 16, and the clamping apparatus 14 may, in response to the second puncture instruction sent by the control device 3, perform puncture along the puncture direction 8 pre-planned by the control device 3 at the puncture position pre-planned by the control device 3, such that the head end of the puncture tool 16 reaches the designated area 7 and forms a puncture wound. Since the instrument such as the working cannula 13 needs to be inserted through the puncture wound in the subsequent operation, the puncture wound may be obtained by the puncture tool 16 puncturing, then reaching the designated area 7 and reaming, obviously, the adopted puncture tool 16 has the function of puncturing and reaming, the puncture tool 16 adopted in the embodiment of the specification may be any existing puncture tool 16 having the function of puncturing and reaming, and the specific material of the puncture tool 16 and the manner of reaming are not limited in the specification.

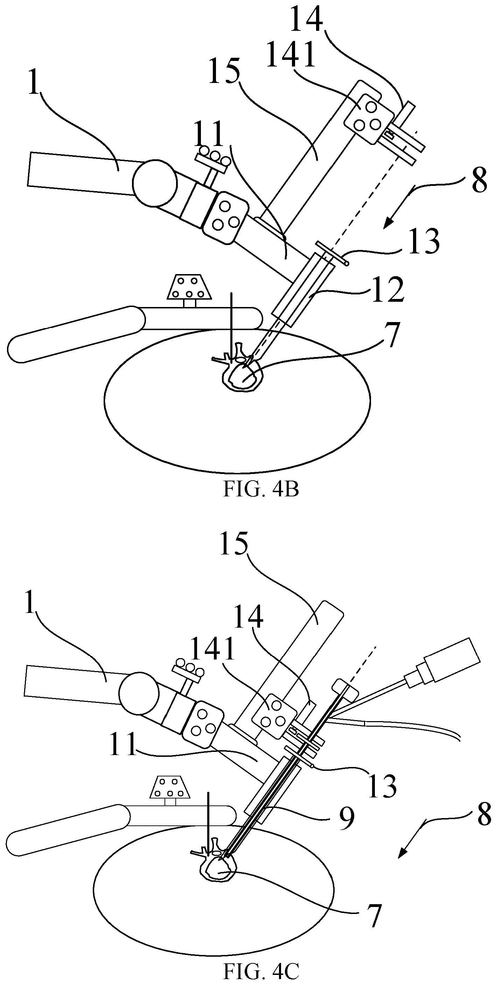

For the puncture wound formed by puncture reaming, the operator can manually place the working cannula 13, as shown in FIG. 4B, the working cannula 13 is inserted into the puncture wound along the puncture direction 8 and secured on the securing apparatus 11, such that the head end of the working cannula 13 is in the designated area 7.

Further, since the securing apparatus 11 further includes a rotating apparatus 12, the rotating apparatus 12 may, in response to a rotation instruction sent by the control device 3, control the rotating apparatus 12 to move, so as to drive the working cannula 13 placed on the securing apparatus 11 to rotate, such that the head end of the working cannula 13 pushes away a dangerous part around the designated area 7 in the puncture wound. The rotation instruction carries a designated rotation direction and a designated rotation angle of the working cannula 13, and the designated rotation direction and the designated rotation angle are determined by the control device 3 according to the designated area 7 and a scanning result of the diseased part.

The timing of the rotation device 12 in response to the rotation instruction sent by the control device 3 may be when the control device 3 detects that the head end of the working cannula 13 is in the designated area 7 before surgery, or may be when an operator in surgery observes and/or the control device 3 detects that the surgical instrument 211 may operate on a dangerous part, which is not limited in the present specification.

Optionally, the first auxiliary mechanical arm 1 may control itself to tilt in response to the tilt instruction sent by the control device 3, so as to drive the working cannula 13 placed on the securing apparatus 11 at the distal end of the first auxiliary mechanical arm 1 to tilt, thereby achieving the purpose of pushing away the dangerous part around the designated area 7. The tilt instruction carries a tilt direction and a tilt angle of the first auxiliary mechanical arm 1, and the tilt direction and the tilt angle of the first auxiliary mechanical arm 1 may be determined by the control device 3 according to relative positions of the first auxiliary mechanical arm 1 and the second auxiliary mechanical arm relative to a designated position of the patient and a scanning result of the diseased part.

For the process of using the clamping apparatus 14 to clamp the endoscope 9 and inserting the endoscope into the puncture wound: as shown in FIG. 4C, after the working cannula 13 is placed and rotated, the tool clamped by the clamping apparatus 14 can be replaced with the endoscope 9 by the puncture tool 16. In response to an endoscope control instruction sent by the control device 3, the clamping apparatus 14 controls the first slide block 141 to slide along the first slide rail 15 to drive the endoscope 9 clamped by the clamping apparatus 14 to be inserted into the working cannula 13, and the endoscope 9 may collect a real-time image of the designated area 7 and send the real-time image of the designated area 7 to the control device 3, such that the control device 3 performs a surgical operation on the designated area 7 according to the real-time image of the designated area 7.

The clamping apparatus 14 may, in response to an endoscope rotation instruction sent by the control device 3, further control the endoscope 9 to rotate, so as to acquire real-time images of the designated area 7 at different angles. Of course, when clamping the endoscope 9, the clamping apparatus 14 may respond to other endoscope instructions sent by the control device 3 according to specific application scenarios and operation actions of the operator, such as controlling the endoscope 9 to move forward and backward.

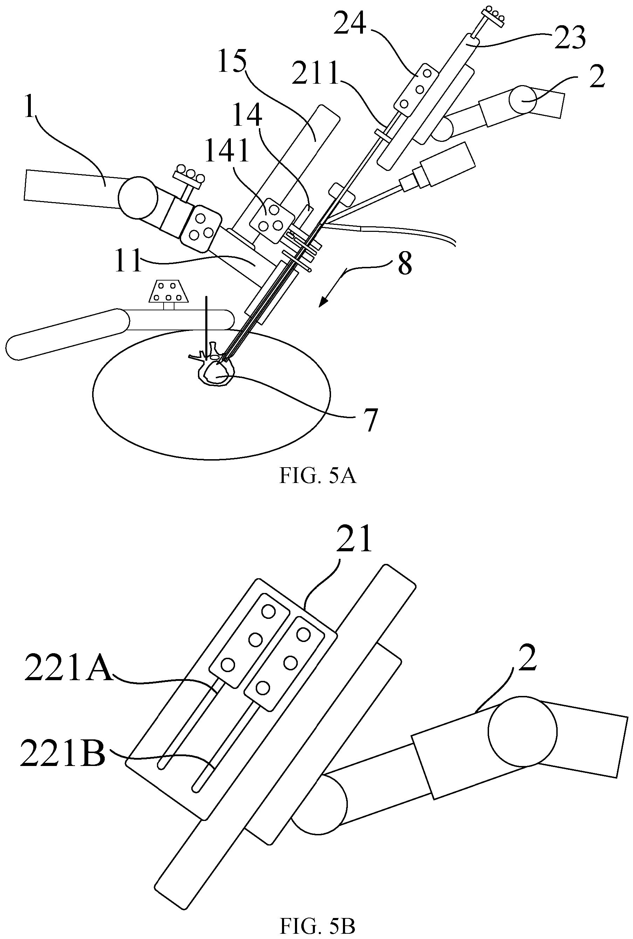

In the embodiments of the specification, the operation mechanical arm 2 may perform the surgical operation on the diseased part of the patient in response to a control instruction sent by the control device 3, as shown in FIG. 5A, specifically including the following steps.

First, the operation mechanical arm 2 includes a second slide rail 23 and a surgical instrument assembly 21, where the second slide rail 23 is fixed on the operation mechanical arm 2. The surgical instrument assembly 21 is configured with a second slide block 24, and the second slide block 24 can slide on the second slide rail 23, and meanwhile, to drive the surgical instrument assembly 21 to slide on the operation mechanical arm 2 along the extending direction of the second slide rail 23, so as to insert the surgical instrument 211 into the working cannula 13 or the working channel of the endoscope 9, and perform the surgical operation on the designated area 7.

Further, when the operation mechanical arm 2 receives a surgical operation instruction sent by the control device 3, the operation mechanical arm 2 controls the surgical instrument 211 included in the surgical instrument assembly 21 to perform the surgical operation on the designated area 7.

The surgical instrument assembly 21 includes a plurality of surgical instruments 211. This is because different surgical instruments are used in the face of different diseases (such as nucleus pulposus, spinal stenosis, nerve root compression, dural sac and bone tissue hyperplasia, etc. ), and a surgical process tailored to the patient may require handling different disease conditions, multiple manual replacements of a single surgical instrument 211 configured on the operation mechanical arm during surgery not only increase surgical time, but also increase the operation difficulty of operator. Therefore, the operator may configure a plurality of surgical instruments 211 in the surgical instrument assembly 21 according to the actual condition of the diseased part of the patient, so as to meet the requirements of using different surgical instruments to perform different surgical operations (such as removing protruding tissue, removing bone, repairing damaged annulus fibrosus by using a radio frequency electrode, and the like) in a surgical procedure.

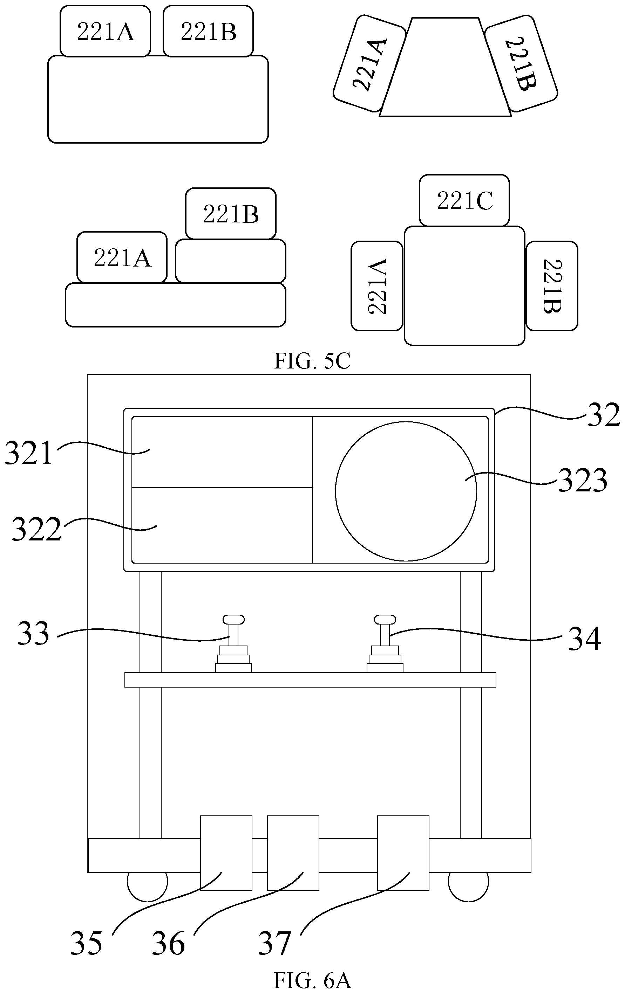

Specifically, when the surgical instrument assembly 21 includes two surgical instruments 211A and 211B, as shown in FIG. 5B. The arrangement of the plurality of surgical instruments 211 included in the surgical instrument assembly 21 is shown in FIG. 5C, and the arrangement of the plurality of surgical instruments 211 may be one of parallel mounting, staggered mounting, inclined surface mounting, zigzag mounting, or the like, where the mounting sequence and the specific position of the surgical instruments 221A, 221B and 221C in the zigzag mounting are not limited in the embodiments of the specification. A plurality of surgical instruments 211 included in the surgical instrument assembly 21 may be arranged by the operator according to specific surgical needs, and a specific surgical instrument 211 arrangement manner is not limited in this embodiment of this specification.

In addition, since the surgical instrument 211 needs to be replaced to perform a surgical operation on the designated area 7 of the patient for different conditions of the diseased part of the patient during the surgical procedure, the surgical instrument assembly 21 may control switching between the surgical instruments 211 included in the surgical instrument assembly 21 in response to the instrument switching instruction sent by the control device 3. The instrument switching instruction sent by the control device 3 may be sent by the operator operating the operation control lever 34 or the operation foot brake 36 configured on the control device 3, or may be sent according to a pre-planned surgical planning action of the surgical instrument 211 on the operation mechanical arm 2 for the diseased part, or may be sent through an instrument switching option configured in the control device 3, which is not limited in the present specification.

In another embodiment of the specification, the control device 3 includes a display 32, an auxiliary control lever 33, and an operation control lever 34, the display 32 may display an image used by an operator to assist the operator in controlling the first auxiliary mechanical arm 1 and the operation mechanical arm 2, and the operator may control the movement of the first auxiliary mechanical arm 1 and the operation mechanical arm 2 by manually operating the operation control lever 34, to implement puncturing, placing and rotating the working cannula 13, surgical operations, and the like. FIG. 6A is a schematic diagram of a control device provided in the specification.

Specifically, the display 32 on the control device 3 is configured to display a scanning result (a CT image or an X-ray image) of the diseased part of the patient, a three-dimensional image generated by the control device 3, and a real-time image of the diseased part in the wound on the body of the patient collected by the endoscope 9, so as to provide a reference for the operator to perform an operation. As shown in FIG. 6A, the scanning result of the diseased part of the patient may be displayed in the display area 321, the three-dimensional image generated by the control device 3 may be displayed in the display area 322, and the real-time image of the diseased part in the wound on the body of the patient collected by the endoscope 9 may be displayed in the display area 323. The three-dimensional image generated by the control device 3 shown in the display area 322 may be a three-dimensional image of the endoscope 9, the surgical instrument 211 and the diseased part of the patient simulated by the control device 3 through the built-in specified three-dimensional software. The operator may, by referring to the image on the display 32, manually control the auxiliary control lever 33 to control the first auxiliary mechanical arm 1 to move , so as to perform puncture, placement and rotation of the working cannula 13 and surgical operation, and manually control the operation control lever 34 to control the operation mechanical arm 2 to move in different directions and control the tip of the surgical instrument 211 to open and close to implement the surgical operation. The operation direction of the auxiliary control lever 33 may include front-back, left-right, and rotation, and the operation direction of the operation control lever 34 may include front-back, left-right, and rotation. Optionally, the operation control lever 34 can be set to move in four directions: forward, backward, left, and right. At this time, the operator can control the operation control lever 34 to move in four directions: forward, backward, left, and right, starting from the current position of the operation control lever 34. The operation control lever 34 can also be used as an axis to control the swinging of the operating control rod 34 in four directions: forward, backward, left, and right.

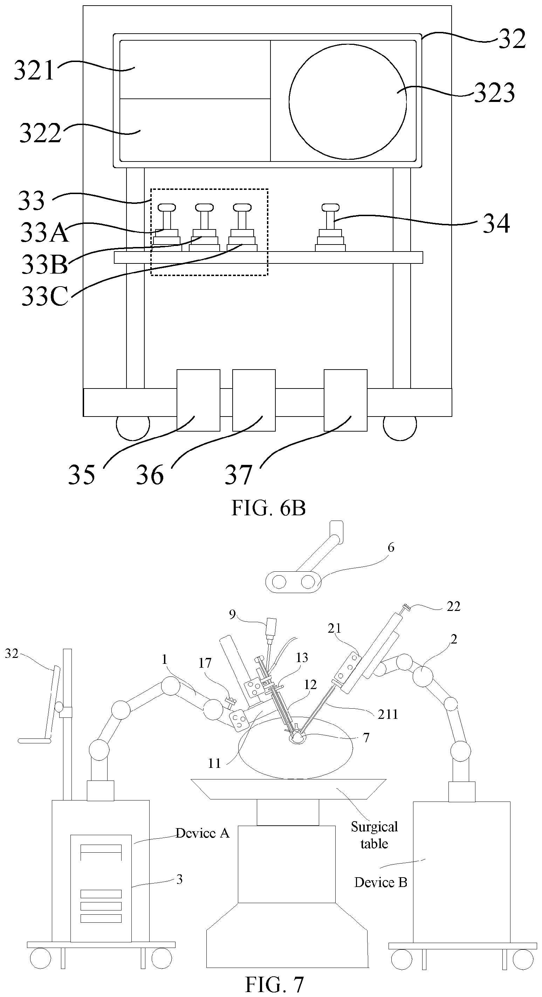

Optionally, the auxiliary control levers may be divided into three auxiliary control levers respectively configured in the control device 3 according to different control functions, as shown in FIG. 6B, the control device 3 is provided with a first auxiliary control lever 33A, a second auxiliary control lever 33B and a third auxiliary control lever 33C. The first auxiliary control lever 33A may be configured to send a rotation instruction to the rotating apparatus 12 of the first auxiliary mechanical arm 1, such that the rotating apparatus 12 drives the working cannula 13 to rotate. The second auxiliary control lever 33B may be configured to send a tilt instruction to the first auxiliary mechanical arm 1, such that the first auxiliary mechanical arm 1 drives the working cannula 13 to tilt. The third auxiliary control lever 33C may be configured to issue control commands such as forward, backward, and rotation to the clamping apparatus 14 on the first auxiliary mechanical arm 1, such that a tool (such as the puncture tool 16 and the endoscope 9) clamped by the clamping apparatus 14 performs forward, backward, and rotation operations.

In addition, an auxiliary foot brake 35, an operation foot brake 36 and an emergency stop foot brake 37 may be provided to cooperate with the operations of the auxiliary control lever 33 and the operation control lever 34.

When the operator depresses the auxiliary foot brake 35, the operator may control the auxiliary control lever 33 to operate the first auxiliary mechanical arm 1, adjust the position of the first auxiliary mechanical arm 1, and fine-adjust the angle and the position of the light source and the camera at the distal end of the endoscope 9. If the operator does not depress the auxiliary foot brake 35, the first auxiliary robotic arm 1 cannot be operated even if the operator operates the auxiliary control lever 33.

When the operator depresses the operation foot brake 36, the operator may control the operation control lever 34 to operate the operation mechanical arm 2, adjust the position of the operation mechanical arm 2 on the body of the patient, implement fine adjustment of the angle and position of the surgical instrument 211, and control the surgical instrument 211 to complete corresponding surgical operations. If the operator does not depress the operation foot brake 36, the operation mechanical arm 2 cannot be operated even if the operator controls the operation control lever 34, to ensure safety.

In addition, the control device 3 is further configured with an emergency stop foot brake 37, and once the operator finds that the first auxiliary mechanical arm 1 and/or the operation mechanical arm 2 has an operation abnormality during execution of the puncture instruction, the rotation instruction, and the surgical operation instruction, and the first auxiliary mechanical arm 1 and/or the operation mechanical arm 2 has an operation abnormality when the operator performs manual operation by using the control device 3, the operator may depress the emergency stop foot brake 37 to stop movements of the first auxiliary mechanical arm 1 and the operation mechanical arm 2.

Of course, the operator can also manually and slightly adjust the angle and position of the endoscope 9 and the surgical instrument 211 arm in the wound according to specific surgical conditions, and manually operate the surgical instrument 211 to perform surgical treatment on the diseased part.

In an alternative embodiment of the specification, an intra-operative CT or C-arm X-ray scan may be performed on a patient during a surgical procedure to obtain an intra-operative X-ray image of a designated region 7 within a diseased part of the patient. Since the scanning of the CT or C-arm X-ray during surgery will cause certain radiation in the surgical environment, in order to protect the body safety of the operator, a protective device may be placed around the control device 3, for example, the protective device may be a ray protective plate or a lead glass device with an equivalent of ≥0.5 mm Pb, thereby preventing the operator from being radiated in the surgical environment.

Optionally, the patient needs to be anesthetized before minimally invasive surgery is performed on the patient. The spinal endoscope minimally invasive surgery in the embodiments of the specification adopts local anesthesia to ensure that the patient is awake during the surgery. This is because the spinal endoscopic minimally invasive surgery is to treat the human tissues such as the disc tissue and the nucleus pulposus protruding in the intervertebral cavity, and the nerve root may be touched during the surgery. The advantage of local anesthesia is that if the surgical operation pulls on the nerve root or there is radio frequency cautery during the operation, which affects the nerve root, the patient can immediately report it, and thus, the operator will stop the operation that may cause damage to the nerve, ensuring the safety of the surgery. In addition, the operator can communicate with the patient at any time during the operation, and the patient is allowed to perform some activities cooperated with the operation such as moving lower limbs, such that the safety of the patient during the operation can be timely confirmed.

Although the local anesthesia can ensure the safety of the patient, since the patient is always awake during the surgical local anesthesia, the body of the patient may be moved, resulting in the deviation of the surgical position, which is a very dangerous condition during the process of assisting the surgical operation by using the surgical device, in order to ensure that the patient does not move during the operation, the second auxiliary mechanical arm 4 can be added to secure the patient, so as to prevent the displacement of the body of the patient.

The surgical device shown in FIG. 1 may further include a second auxiliary mechanical arm 4, and the second auxiliary mechanical arm 4 may be mounted on the device A where the first auxiliary mechanical arm 1 is located, or may be mounted on one side of the surgical bed, the device B, or a separate device. When it is determined that the patient is at a designated position on the patient bed, the second auxiliary mechanical arm 4 may press the patient to secure the diseased part of the patient, so as to prevent injury to the patient due to displacement of the diseased part of the patient during surgery. In addition, since the flexibility requirement of the second auxiliary mechanical arm 4 is not high, the second auxiliary mechanical arm 4 may be a mechanical arm with three or more movable joints.

Further, in order to prevent the diseased part of the patient from moving during the operation, the second auxiliary mechanical arm 4 may, in response to the first puncture instruction sent by the control device 3, control the securing needle 41 configured at the distal end of the second auxiliary mechanical arm 4 to puncture the body of the patient, so as to further secure the diseased part of the patient.

Optionally, the second auxiliary mechanical arm 4 may be manually controlled by an operator to move and press the patient with the end of the second auxiliary mechanical arm 4 to secure the patient.

Alternatively, the operator may manually puncture the patient to implant the securing needle 41.

Optionally, the distal end of the second auxiliary mechanical arm 4 is provided with a third positioning light source mark 42, the third positioning light source mark 42 is configured to indicate a position of a diseased part of the patient, and the third positioning light source mark 42 is combined with the first positioning light source mark 22 and the second positioning light source mark 17 to jointly determine relative positions of the first auxiliary mechanical arm 1 and the operating mechanical arm 2 relative to the patient, such that accuracy of positioning the positions of the first auxiliary mechanical arm 1 and the operating mechanical arm 2 can be improved.

In addition, the third positioning light source mark 42 is further configured to indicate the movement (displacement) of the body of the patient, although the second auxiliary mechanical arm 4 and the securing needle configured at the distal end of the second auxiliary mechanical arm 4 have been used to secure the diseased part of the patient, considering the uncertainty of the movement of the diseased part of the patient during the operation, the positioning device 6 may track the micro-motion of the body of the patient through the light source emitted or reflected by the third positioning light source mark 42, so as to adjust the control of the first auxiliary mechanical arm 1 and the operation mechanical arm 2 in real time when the patient's body is moved (displaced).

Based on this, in order to enable the third positioning light source mark 42 to have a function of indicating the position of the diseased part of the patient, the placement position of the second auxiliary mechanical arm 4 where the third positioning light source mark 42 is located on the body of the patient needs to be located at a position within a preset range of the diseased part of the patient, for example, during the minimally invasive spinal surgery, in order to prevent the second auxiliary mechanical arm 4 from affecting the surgical operation on the diseased part, the operator places the second auxiliary mechanical arm 4 at a position within the preset range of the diseased part of the patient, and enables the securing needle 41 configured at the distal end of the second auxiliary mechanical arm 4 to be inserted into other bone tissue (vertebral body or vertebral pedicle) within the preset range near the diseased spine of the patient. In this way, the second auxiliary mechanical arm 4 can be prevented from affecting the puncture operation performed by the puncture tool 16 on the first auxiliary mechanical arm 1 on the body of the patient, and the surgical operation of the operation mechanical arm 2 for the diseased part of the patient, and the functions of securing the body of the patient and indicating the position of the diseased part of the patient can also be achieved. The preset range may be set according to actual conditions, which is not limited in the present disclosure.

Optionally, the specification further provides a surgical device for UBE surgery by a first auxiliary mechanical arm mounting with an endoscope and an operation mechanical arm mounting with a surgical instrument, as shown in FIG. 7. A working cannula 13 is secured on the securing apparatus 11 at the distal end of the first auxiliary mechanical arm 1, and the endoscope 9 enters from the working cannula 13 to collect a real-time image of a designated area of the patient and sends the real-time image to the control device 3. The surgical instrument assembly 21 is configured at the distal end of the operation mechanical arm 2, enters the patient from another puncture wound different from the puncture wound where the working cannula 13 is located and reaches the designated area, and performs, in response to a pre-planned surgical action or an operation action performed by an operator on the control device, a surgical operation on the designated area.

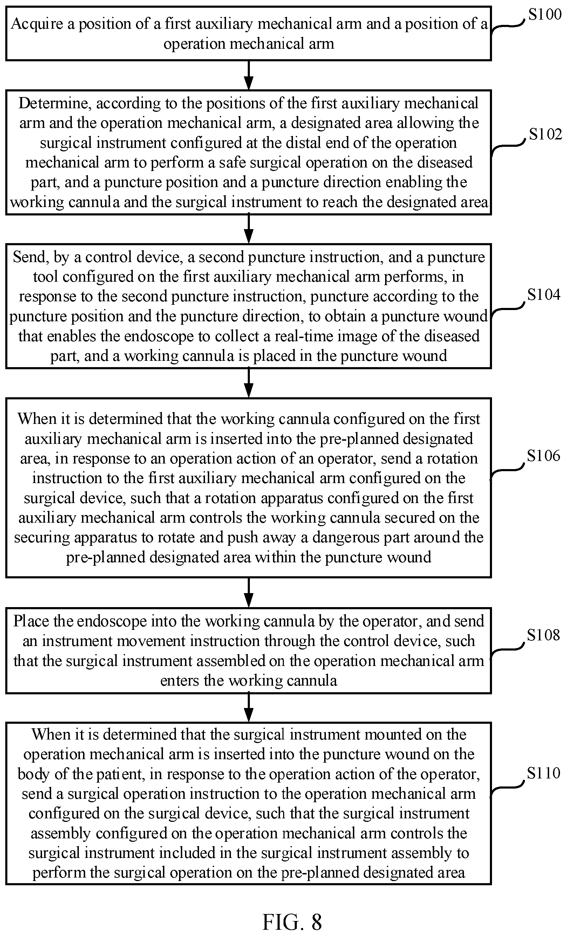

To facilitate understanding of the foregoing surgical device, the specification further provides a schematic flowchart of a surgical method, to describe a control method of the foregoing surgical device in an actual surgical procedure, as shown in FIG. 8.

FIG. 8 is a flowchart of a minimally invasive surgical device operation method provided in the specification, including the following steps.

S100: a position of the first auxiliary mechanical arm and a position of the operation mechanical arm are acquired.

Generally, the control device may be a computer host installed with specified software (such as three-dimensional software, planning software, etc.), which is connected to the first auxiliary mechanical arm, the operation mechanical arm and other devices in a wired or wireless manner. Therefore, the surgical method provided in the embodiments of the specification can be executed by a control device executing the execution process of the surgical method.

The positioning device acquires the position of the first auxiliary mechanical arm and the position of the operation mechanical arm by receiving the light emitted or reflected by the auxiliary positioning light source marks, which include the first auxiliary positioning light source mark 22 mounted on the first auxiliary mechanical arm, the second auxiliary positioning light source mark 17 mounted on the operating mechanical arm and a third auxiliary positioning light source mark mounted on the second auxiliary positioning light source mark.

The positioning device sends the acquired positions of the first auxiliary mechanical arm and the operation mechanical arm to the control device, and the control device receives and displays the positions of the first auxiliary mechanical arm and the operation mechanical arm.

Optionally, the diseased part is scanned by the CT or C-arm X-ray, and the control device in combination with the scanning result and the positions of the first auxiliary mechanical arm and the operation mechanical arm, the relative positions of the first auxiliary mechanical arm and the operation mechanical arm relative to the diseased part are determined.

S102: according to the positions of the first auxiliary mechanical arm and the operation mechanical arm, a designated area allowing the surgical instrument configured at the distal end of the operation mechanical arm to perform a safe surgical operation on the diseased part, and a puncture position and a puncture direction enabling the working cannula and the surgical instrument to reach the designated area are determined.

The planning module in the control device may determine, according to the relative positions of the first auxiliary mechanical arm 1 and the operating mechanical arm relative to the patient and the scanning result of the diseased part of the patient, the designated area allowing the surgical instrument configured at the distal end of the operation mechanical arm to perform a safe surgical operation on the diseased part, and the puncture position and the puncture direction enabling the working cannula and the surgical instrument to reach the designated area. The puncture position and the puncture direction enabling the surgical instrument to reach the designated area can be used as a surgical path planned by the control device for the diseased part of the patient.

S104: the control device sends a second puncture instruction, and a puncture tool configured on the first auxiliary mechanical arm performs, in response to the second puncture instruction, puncture according to the puncture position and the puncture direction, to obtain a puncture wound that enables the endoscope to collect a real-time image of the diseased part, and a working cannula is placed in the puncture wound.

When the patient is at the designated position and the first auxiliary mechanical arm swings to the above side of the patient, the clamping apparatus clamps the puncture tool, and the clamping apparatus can respond to the second puncture instruction sent by the control device and perform puncture at the puncture position planned in advance by the control device along the puncture direction planned in advance by the control device, such that the head end of the puncture tool reaches the designated area and forms a puncture wound.

For a puncture wound formed by puncture reaming, a working cannula can be manually placed by an operator, the working cannula is inserted into the puncture wound along the puncture direction, and the working cannula is secured on the securing apparatus, such that the head end of the working cannula is in the designated area.

S106: when it is determined that the working cannula configured on the first auxiliary mechanical arm is inserted into the pre-planned designated area, in response to an operation action of an operator, a rotation instruction is sent to the first auxiliary mechanical arm configured on the surgical device, such that a rotation apparatus configured on the first auxiliary mechanical arm controls the working cannula secured on the securing apparatus to rotate and push away a dangerous part around the pre-planned designated area within the puncture wound.

Further, the securing apparatus further includes a rotating apparatus, and the rotating apparatus can control the rotating apparatus to move in response to a rotating instruction sent by the control device, so as to drive the working cannula placed (disposed) on the securing apparatus to rotate, such that the head end of the working cannula pushes away the dangerous part around the designated area in the wound. The rotation instruction carries a designated rotation direction and a designated rotation angle of the working cannula, and the designated rotation direction and the designated rotation angle are determined by the control device according to the designated area and the scanning result of the diseased part.

S108: the endoscope is placed into the working cannula by the operator, and an instrument movement instruction is sent through the control device, such that the surgical instrument assembled on the operation mechanical arm enters the working cannula.

After the working cannula is placed and rotated, the tool clamped by the clamping apparatus can be replaced with the endoscope by the puncture tool. The clamping apparatus responds to the endoscope control instruction sent by the control device, controls the first slide block to slide along the first slide rail to drive the endoscope clamped by the clamping apparatus to be inserted into the working cannula, and the endoscope can collect the real-time image of the designated area and send the real-time image of the designated area to the control device, such that the control device performs surgical operation on the designated area according to the real-time image of the designated area.

S110: when it is determined that the surgical instrument mounted on the operation mechanical arm is inserted into the puncture wound on the body of the patient, in response to an operation action of the operator, a surgical operation instruction is sent to the operation mechanical arm configured on the surgical device, such that the surgical instrument assembly configured on the operation mechanical arm controls the surgical instrument included in the surgical instrument assembly to perform the surgical operation on the pre-planned designated area.

It can be seen that by automatically rotating the working cannula by the first auxiliary mechanical arm, the human tissue in the dangerous area can be protected, thereby reducing the risk of intraoperative infection and postoperative complications, and eliminating the manual rotation operation performed by the operator during the surgery.

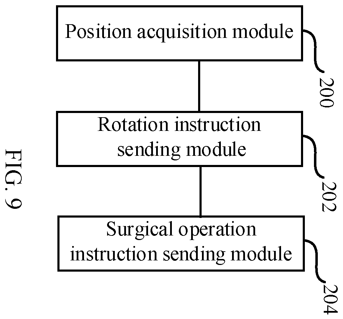

The above are the surgical methods provided in one or more embodiments of the specification. Based on the same idea, the specification further provides a corresponding surgical apparatus, as shown in FIG. 9.

FIG. 9 is a schematic diagram of a surgical apparatus according to the specification, specifically including:
a position acquisition module 200, configured to acquire a position of the first auxiliary mechanical arm and a position of the operation mechanical arm;
a rotation instruction sending module 202, configured to: in response to determining that a working cannula configured on the first auxiliary mechanical arm is inserted into a pre-planned designated area, send, in response to an operation action of an operator, a rotation instruction to the first auxiliary mechanical arm configured on any one of the foregoing surgical devices, such that a rotation apparatus configured on the first auxiliary mechanical arm controls a working cannula secured on the securing apparatus to rotate and push away the dangerous part around the pre-planned designated area in a puncture wound; and
a surgical operation instruction sending module 204, configured to: in response to determining that a surgical instrument configured on the operation mechanical arm is inserted into the puncture wound on the body of the patient, send, in response to the operation action of the operator, a surgical operation instruction to the operation mechanical arm configured on any one of the foregoing surgical devices, such that a surgical instrument assembly configured on the operation mechanical arm controls a surgical instrument included in the surgical instrument assembly to perform a surgical operation on the pre-planned designated area.

The specification further provides a computer-readable storage medium storing computer programs, when executed by a processor, causing the processor to implement the above-mentioned surgical method.

Of course, in addition to the software implementation, other embodiments, such as a logic device or a combination of software and hardware, are not excluded in the specification, that is, the execution body of the following processing flow is not limited to each logic unit, and may also be hardware or a logic device.

In the 1990s, for a technological improvement, there was a clear distinction between an improvement in hardware (for example, for the circuit structure of diodes, transistors, switches, etc.) and an improvement in software (for methods and processes). However, with the development of technology, the improvement of many methods and processes can be regarded as a direct improvement of the structure of a hardware circuit. Designers almost always obtain the corresponding hardware circuit structure by programming the improved method or process into a hardware circuit. Therefore, it is possible that an improvement of a method or a process is realized by entity modules of hardware. For example, a Programmable Logic Device (PLD) (for example, a Field Programmable Gate Array (FPGA)), is such an integrated circuit whose logic function is determined by the user programming the device. A digital system is "integrated" on a PLD through the programming of the designers, rather than a dedicated integrated circuit chip designed and produced by a chip manufacturer. Moreover, nowadays, instead of making integrated circuit chips manually, this programming is mostly implemented using "logic compiler" software, which is similar to the software compiler used in program development, and the original code before compilation must be written in a specific programming language, which is called a Hardware Description Language (HDL). There are multiple HDLs rather than one, such as ABEL (Advanced Boolean Expression Language), AHDL (Altera Hardware Description Language), Confluence, CUPL (Cornell University Programming Language), HDCal, JHDL (Java Hardware Description Language), Lava, Lola, MyHDL, PALASM, RHDL (Ruby Hardware Description Language), etc., and the most commonly used currently are VHDL (Very-High-Speed Integrated Circuit Hardware Description Language) and Verilog. A person of ordinary skill in the art should also understand that by logically programming the method or process using the above-mentioned several hardware description languages and integrated it into an integrated circuit, a hardware circuit that implements such logic method or process can be easily obtained.

The controller may be implemented in any suitable manner. For example, the controller may take the form of a microprocessor or processor and a computer-readable medium, logic gates, switches, application specific integrated circuits (ASICs), programmable logic controllers, and embedded microcontrollers storing computer-readable program code (such as software or firmware) executable by the (micro) processor. Examples of the controller include, but are not limited to, the following microcontrollers: ARC 625D, Atmel AT91SAM, Microchip PIC18F26K20 and Silicone Labs C8051F320. The memory controller can also be implemented as part of the control logic for the memory. A person of ordinary skill in the art also knows that, in addition to implementing the controller in pure computer-readable program codes, it is entirely possible to make the controller logic gates, switches, dedicated integrated circuits, programmable logic controllers, embedded microcontrollers and the like to achieve the same function by logically programming the method and the steps. Therefore, such a controller can be regarded as a hardware component, and apparatuses included in the controller for implementing various functions can also be regarded as structures within the hardware component. Or even, devices for implementing various functions may be regarded as both software modules implementing the method and structures within hardware component.

The system, apparatus, module, or unit described in the above-mentioned embodiments can be implemented by a computer chip or entity, or can be implemented by using a product with a certain function. A typical implementation device is a computer. The computer may be a personal computer, a laptop computer, a cellular phone, a camera phone, a smartphone, a personal digital assistant, a media player, a navigation device, an email device, a game console, a tablet computer, a wearable device, or any combination of these devices.

For convenience of description, when describing the above apparatus, the functions are divided into various units and described separately. Of course, when implementing the present disclosure, the functions of the units may be implemented in the same software or multiple softwares and/or hardwares.

A person of ordinary skill in the art should understand that the embodiments of the present disclosure may be provided as a method, a system, or a computer program product. Therefore, the present disclosure may take the form of an entirely hardware implementation, an entirely software implementation, or an implementation combining both software and hardware aspects. Moreover, the present disclosure may take the form of a computer program product implemented on one or more computer-usable storage media (including, but not limited to, disk memory, CD-ROM, optical memory, etc.) containing computer-usable program code.

The present disclosure is described with reference to flowcharts and/or block diagrams of methods, devices (systems), and computer program products according to embodiments of the present disclosure. It should be understood that each process and/or block in the flowcharts and/or block diagrams, and combinations of processes and/or blocks in the flowcharts and/or block diagrams can be implemented by computer program instructions. These computer program instructions may be provided to a processor of a general-purpose computer, a special-purpose computer, an embedded processor, or other programmable data processing devices to produce a machine, such that through instructions executed by the processor of the computers or other programmable data processing devices, an apparatus for implementing the functions specified in one or more processes of the flowcharts and/or one or more blocks of the block diagrams is produced.

These computer program instructions may also be stored in a computer-readable memory capable of directing a computer or other programmable data processing device to work in a specific manner such that the instructions stored in the computer-readable memory produce a manufactured article including an instruction device, where the instruction device implements the functions specified in one or more processes of the flowcharts and/or one or more blocks of the block diagrams.

These computer program instructions can also be loaded onto a computer or other programmable data processing device, such that a series of steps can be performed on the computer or other programmable device to produce a computer-implemented process, and the instructions executed in the computers or other programmable data processing devices provide steps for implementing the functions specified in one or more processes of the flowcharts and/or one or more blocks of the block diagrams.

In a typical configuration, a computing device includes one or more processors (CPUs), an input/output interface, a network interface, and a memory.

The memory may include a non-permanent memory, a random access memory (RAM), and/or a non-volatile memory in computer-readable media, such as a read-only memory (ROM) or a flash RAM. Memory is an example of the computer readable medium.

Computer-readable media includes permanent and non-persistent, removable and non-removable media. Information storage can be accomplished by any method or technology. Information can be computer readable instructions, data structures, modules of a program, or other data. Examples of computer storage medium include, but not limited to, a phase change memory (PRAM), a static random access memory (SRAM), a dynamic random access memory (DRAM), other types of random access memory (RAM), a read only memory (ROM), an electrically erasable programmable read only memory (EEPROM), a flash memory or other memory technologies, a compact disk read only memory (CD-ROM), a digital versatile disk (DVD) or other optical storage, a cassette tape, a disk tape storage or other magnetic storage devices or any other non-transportable medium, which may be used to store information that may be accessed by a computing device. Based on the definition in the present disclosure, computer-readable media does not include transitory computer-readable media (transitory media), such as modulated data signals and carrier waves.

It should also be noted that the terms "including", "comprising" or any other variation are intended to cover non-exclusive inclusion, such that a process, a method, a product or a device that includes a series of elements includes not only those elements, but also includes other elements that are not explicitly listed, or elements that are inherent to such process, method, product, or device. Without more restrictions, the elements defined by the sentence "including a ..." do not exclude the existence of other identical elements in the process, method, product or device including the elements.

Those skilled in the art should understand that the examples of the present disclosure can be implemented as a method, a system, or a computer program product. Therefore, the present disclosure takes the form of an entirely hardware implementation, an entirely software implementation or an implementation combining both software and hardware aspects. Moreover, the present disclosure may take the form of a computer program product implemented on one or more computer-usable storage media (including, but not limited to, disk memory, CD-ROM, optical memory, etc.) containing computer-usable program code.

This disclosure can be described in the general context of computer-executable instructions executed by a computer, such as program modules. Generally, program modules include routines, programs, objects, components, data structures, etc. that perform specific tasks or implement specific abstract data types. The present disclosure can also be practiced in distributed computing environments in which tasks are performed by remote processing devices connected through a communication network. In a distributed computing environment, program modules may be located in local and remote computer storage media, including storage devices.

Each embodiment in the present disclosure is described in a progressive manner, and the same or similar parts between the various embodiments can be referred to each other. Each embodiment focuses on the differences from other embodiments. In particular, with respect to the system embodiments, since they are basically similar to the method embodiments, the description thereof is relatively simple. For the related parts, reference may be made to the description of the method embodiments.

The above are only implementations of the present disclosure and are not intended to limit the specification. For those skilled in the art, the specification may have various modifications and changes. Any modification, equivalent replacement, and improvement made within the spirit and principle of the specification shall be included in the scope of claims of this disclosure.

## Claims

1. A surgical device, **characterized in that**, the surgical device comprises a first auxiliary mechanical arm (1), an operation mechanical arm (2), and a control device (3);
wherein the control device (3) is configured to acquire a position of the first auxiliary mechanical arm (1) and a position of the operation mechanical arm (2), and send control instructions to the first auxiliary mechanical arm (1) and the operation mechanical arm (2) according to the position of the first auxiliary mechanical arm (1), the position of the operation mechanical arm (2) and an operation action of an operator;
the first auxiliary mechanical arm (1) comprises a securing apparatus (11) and a rotating apparatus (12); the rotating apparatus (12) is configured on the securing apparatus (11); the securing apparatus (11) is used for placing a working cannula (13) inserted into a patient; and the first auxiliary mechanical arm (1) is configured to control, in response to a control instruction sent by the control device (3), the working cannula (13) placed on the securing apparatus (11) to rotate and push away a dangerous part around a pre-planned designated area (7) in a puncture wound; and
the operation mechanical arm (2) is configured with a surgical instrument assembly (21); and the operation mechanical arm (2) is configured to control, in response to a control instruction sent by the control device (3), a surgical instrument (211) comprised in the surgical instrument assembly (21) to perform a surgical operation on the designated area (7).

2. The surgical device according to claim 1, further comprises a second auxiliary mechanical arm (4); wherein the second auxiliary mechanical arm (4) comprises a securing needle (41);
in response to determining that the patient is at a designated position, the control device (3) is configured to control the second auxiliary mechanical arm (4) to press the patient; and
the second auxiliary mechanical arm (4) is configured to control, in response to a first puncture instruction sent by the control device (3), the securing needle (41) configured at a distal end of the second auxiliary mechanical arm (4) to puncture a body of the patient, so as to secure a diseased part of the patient.

3. The surgical device according to claim 1, further comprises a second auxiliary mechanical arm (4); wherein the second auxiliary mechanical arm (4) comprises a securing needle (41);
when the patient is at a designated position, the second auxiliary mechanical arm (4) is manually controlled by the operator to press the patient; and
the securing needle (41) configured at a distal end of the second auxiliary mechanical arm (4) is manually controlled by the operator to puncture the patient, so as to secure a diseased part of the patient.

4. The surgical device according to claim 1, further comprises a scanning device (5); wherein the scanning device (5) is configured to scan the patient and obtain a scanning result of a diseased part of the patient; and
the scanning device (5) is configured to send the scanning result of the diseased part to the control device (3), such that the control device (3) receives and displays the scanning result of the diseased part.

5. The surgical device according to claim 1, further comprises a positioning device (6); wherein the operation mechanical arm (2) is configured with a first positioning light source mark (22); the first auxiliary mechanical arm (1) is configured with a second positioning light source mark (17); a second auxiliary mechanical arm (4) is configured with a third positioning light source mark (42); and
the positioning device (6) is configured to determine a relative position of the operation mechanical arm (2) relative to the patient and a relative position of the first auxiliary mechanical arm (1) relative to the patient according to collected light rays emitted or reflected by the first positioning light source mark (22), the second positioning light source mark (17) and the third positioning light source mark (42), and send the relative positions to the control device (3), such that the control device (3) sends the control instructions to the first auxiliary mechanical arm (1) and the operation mechanical arm (2) according to the position of the operation mechanical arm (2) and the position of the first auxiliary mechanical arm (1) and the operation action of the operator.

6. The surgical device according to claim 1, wherein the control device (3) comprises a planning module (31); the planning module (31) is configured to determine the designated area (7), a puncture position and a puncture direction (8) according to the positions of the operation mechanical arm (2) and the first auxiliary mechanical arm (1) and a scanning result of a diseased part of the patient; wherein the designated area (7) is an area allowing the surgical instrument (211) configured at a distal end of the operation mechanical arm (2) to perform an safe surgical operation on a diseased part of the patient; and the puncture position and the puncture direction (8) are used for enabling the working cannula (13) and the surgical instrument (211) to reach the designated area (7), such that the control device (3) controls the first auxiliary mechanical arm (1) and the operation mechanical arm (2) to perform the surgical operation on the designated area according to the designated area (7), the puncture position and the puncture direction (8).

7. The surgical device according to claim 1, wherein the first auxiliary mechanical arm (1) comprises a first slide rail (15) and a clamping apparatus (14); wherein the first slide rail (15) is fixed on the first auxiliary mechanical arm (1); the clamping apparatus (14) is configured with a first slide block (141), and the first slide block (141) is configured to slide on the first slide rail (15) to drive the clamping apparatus (14) to slide on the first auxiliary mechanical arm (1) along an extension direction of the first slide rail (15), so as to drive a tool clamped by the clamping apparatus (14) to slide along the extension direction of the first slide rail (15).

8. The surgical device according to claim 7, wherein the clamping apparatus (14) is configured to clamp a puncture tool (16); the clamping apparatus (14) is configured to control, in response to a second puncture instruction sent by the control device (3), the first slide block (141) to slide along the puncture direction (8) on the first slide rail (15), so as to drive the puncture tool (16) to puncture along the puncture direction (8) at the puncture position, such that a head end of the puncture tool (16) reaches the designated area (7) to form the puncture wound.

9. The surgical device according to claim 8, wherein the working cannula (13) is inserted into the puncture wound along the puncture direction (8) and secured on the securing apparatus (11), such that a head end of the working cannula (13) is in the designated area (7); and
in response to detecting that the head end of the working cannula (13) is in the designated area (7), the rotating apparatus (12) is controlled to rotate in response to a rotating instruction sent by the control device (3) and drive the working cannula (13) placed on the securing apparatus (11) to rotate, such that the head end of the working cannula (13) pushes away a dangerous part around the designated area (7) in the puncture wound.

10. The surgical device according to claim 9, wherein the rotation instruction carries a designated rotation direction and a designated rotation angle of the working cannula (13); and the designated rotation direction and the designated rotation angle are determined by the control device (3) according to the designated area (7) and a scanning result of the diseased part.

11. The surgical device according to claim 7, wherein the clamping apparatus (14) is configured to clamp an endoscope (9); the clamping apparatus (14) is configured to control, in response to an endoscope control instruction sent by the control device (3), the first slide block (141) to slide along the first slide rail (15) to drive the endoscope (9) clamped by the clamping apparatus (14) to be inserted into the working cannula (13), so as to collect and send a real-time image of the designated area (7).

12. The surgical device according to claim 1, wherein the operation mechanical arm (2) comprises a second slide rail (23) and a surgical instrument assembly (21); wherein the second slide rail (23) is fixed on the operation mechanical arm (2); the surgical instrument assembly (21) is configured with a second slide block (24), and the second slide block (24) is configured to slide on the second slide rail (23) to drive the surgical instrument assembly (21) to slide on the operation mechanical arm (2) along an extension direction of the second slide rail (23), so as to insert the surgical instrument (211) into the working cannula (13) or a working channel of an endoscope (9) to perform the surgical operation on the designated area (7).

13. The surgical device according to claim 1 or 12, wherein the surgical instrument assembly (21) comprises a plurality of surgical instruments (211); the plurality of surgical instruments (211) are mounted in the surgical instrument assembly (21) in a form comprising at least one of: parallel mounting, staggered mounting, inclined surface mounting and zigzag mounting; and
the surgical instrument assembly (21) is configured to control, in response to an instrument switching instruction sent by the control device (3), switching between surgical instruments (211) comprised in the surgical instrument assembly (21).

14. The surgical device according to claim 1, wherein the control device (3) further comprises a display (32), an auxiliary control lever (33) and an operation control lever (34);
wherein the display (32) is configured to display a real-time image of the designated area (7) and a scanning result of a diseased part of the patient;
the auxiliary control lever (33) is configured to send a rotation instruction to the rotating apparatus (12) in response to a first operation action of the operator, such that the rotating apparatus (12) rotates to drive the working cannula (13) placed on the securing apparatus (11) to rotate and push away the dangerous part around the designated area (7) in the puncture wound; and
the operation control lever (34) is configured to, in response to a second operation action of the operator, send an instrument movement instruction to the operation mechanical arm (2) and control a second slide block (24) configured on the surgical instrument assembly (21) to slide along a second slide rail (23) to drive a surgical instrument (211) comprised in the surgical instrument assembly (21) to be inserted into the working cannula (13) along a puncture direction (8); and in response to detecting that the surgical instrument (211) reaches the designated area (7), the operation control lever (34) is configured to send a surgical operation instruction to the surgical instrument (211) in response to a third operation action of the operator, so as to control the surgical instrument (211) to perform the surgical operation on the designated area (7).

15. A surgical method, **characterized in that**, the surgical method applied to a control device, wherein the method comprises:
acquiring a position of a first auxiliary mechanical arm and a position of an operation mechanical arm;
in response to determining that a working cannula configured on the first auxiliary mechanical arm is inserted into a pre-planned designated area, in response to an operation action of an operator, sending a rotation instruction to the first auxiliary mechanical arm configured on the surgical device according to any one of claims 1 to 14, such that a rotating apparatus configured on the first auxiliary mechanical arm controls the working cannula secured on the securing apparatus to rotate and push away the dangerous part around the pre-planned designated area within the puncture wound; and
in response to determining that the surgical instrument mounted on the operation mechanical arm is inserted into the puncture wound on a body of the patient, in response to the operation action of the operator, sending a surgical operation instruction to the operation mechanical arm configured on the surgical device according to any one of claims 1 to 14, such that a surgical instrument assembly configured on the operation mechanical arm controls the surgical instrument comprised in the surgical instrument assembly to perform the surgical operation on the pre-planned designated area.

16. A surgical apparatus, **characterized in that**, the surgical apparatus applied to a control device, wherein the surgical apparatus comprises:
a position acquisition module, configured to acquire a position of the first auxiliary mechanical arm and a position of the operation mechanical arm;
a rotation instruction sending module, configured to: in response to determining that a working cannula configured on the first auxiliary mechanical arm is inserted into a pre-planned designated area, send, in response to an operation action of an operator, a rotation instruction to the first auxiliary mechanical arm configured on the surgical device according to any one of claims 1 to 14, such that a rotation apparatus configured on the first auxiliary mechanical arm controls the working cannula secured on the securing apparatus to rotate and push away the dangerous part around the pre-planned designated area in the puncture wound; and
a surgical operation instruction sending module, configured to: in response to determining that a surgical instrument mounted on the operation mechanical arm is inserted into the puncture wound on a body of a patient, send, in response to the operation action of the operator, a surgical operation instruction to the operation mechanical arm configured on the surgical device according to any one of claims 1 to 14, such that a surgical instrument assembly configured on the operation mechanical arm controls the surgical instrument comprised in the surgical instrument assembly to perform a surgical operation on the pre-planned designated area.

17. A computer-readable storage medium configured with computer programs executable by a processor to cause the processor to perform the method of claim 15.
